# EUROPÄISCHE PATENTSCHRIFT

(11) **EP 2 376 550 B1**
(45) Veröffentlichungstag und Bekanntmachung des Hinweises auf die Patenterteilung: **02.04.2014**
(21) Anmeldenummer: 09760906.9
(22) Anmeldetag: 02.12.2009
(51) Int. Cl.: C08F 226/10, C08F 220/30, C08F 216/14, C09D 139/04, C09J 139/04, A61K 8/81, B01D 67/00, A61Q 19/00

(54) **MODIFIZIERTE POLYVINYLLACTAME**
MODIFIED POLYVINYLLACTAMS
POLYVINYLLACTAMES MODIFIÉS

(30) Priorität: 12.12.2008 EP 08171502
(43) Veröffentlichungstag der Anmeldung: 19.10.2011
(73) Patentinhaber: BASF SE, 67056 Ludwigshafen (DE)
(72) Erfinder: FISCHER, Frank, 67281 Kirchheim (DE); WEGMANN, Ludger, 67063 Ludwigshafen (DE); EHLE, Michael, 67071 Ludwigshafen (DE); BAUER, Stephan, 65239 Hochheim (DE); MEYER, Harald, 67157 Wachenheim (DE); NGUYEN KIM, Son, 69502 Hemsbach (DE)
(86) Internationale Anmeldenummer: PCT/EP2009/066188
(87) Internationale Veröffentlichungsnummer: WO 2010/066613

(56) Entgegenhaltungen:
- EP-A2- 0 377 191
- EP-A2- 0 377 199
- WO-A1-03/064061
- WO-A1-03/070792
- WO-A2-99/47176
- GB-A- 2 109 392
- US-A- 3 992 356

## Beschreibung

Gegenstand der vorliegenden Erfindung ist ein Copolymerisat, enthaltend in einpolymerisierter Form

| | |
|---|---|
| 0,01 bis 20 Gew.-% | wenigstens einer ethylenisch ungesättigten Verbindung der allgemeinen Formel (I) [Monomere A], |
| 50 bis 99,99 Gew.-% | wenigstens eines N-Vinyllactams der allgemeinen Formel (VII) [Monomere B] und |
| 0 bis 49,99 Gew.-% | wenigstens einer weiteren, sich von den Monomeren A und Monomeren B unterscheidenden ethylenisch ungesättigten Verbindung [Monomere C], wobei sich die Gesamtmengen der Monomeren A, B und C zu 100 Gew.-% aufsummieren (Gesamtmonomerenmenge), mit |

R-(C=O)-R¹ als Formel (I), wobei
- R: für einen geradkettigen C₁-C₄-Alkylrest, einen verzweigten, gegebenenfalls substituierten C₃- bzw. C₄-Alkylrest, einen Arylrest, oder den Rest R¹ steht und
- R¹: für den Rest 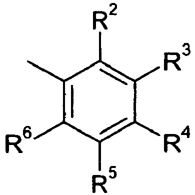
steht, wobei die Reste R² bis R⁶ untereinander gleich oder verschieden sind und für Wasserstoff, Alkyl, Aryl, OH, OCH₃, OC₂H₅, SH, SCH₃, SC₂H₅, F, Cl, Br, CN, CO₂H, CO₂Alkyl, CO₂Aryl, CF₃, N(Alkyl)₂, N(Alkyl)(Aryl), N(Aryl)₂, N⁺(Alkyl)₃A⁻, N⁺H(Alkyl)₂A⁻, wobei A⁻ für das Anion einer Säure steht und mindestens einer aber maximal drei der Reste R² bis R⁶ für einen der Reste 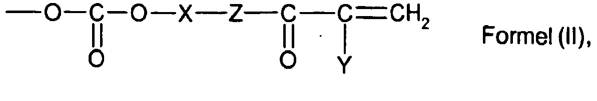 oder 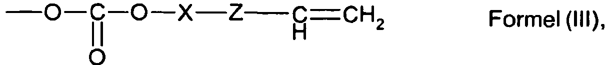 oder 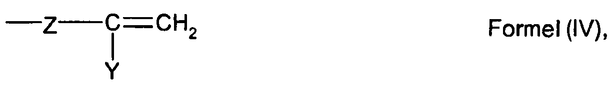 oder 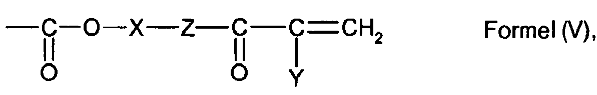 oder 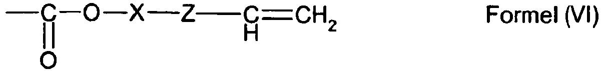 stehen, worin
- X: für einen zweiwertigen, gegebenenfalls substituierten Alkylenrest -(CH₂)ₘ-, einen Rest 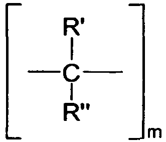
mit m = 1 bis 10, worin R' und R" untereinander gleich oder verschieden sind und für Wasserstoff, Alkyl, Aryl, CO₂H, CO₂CH₃ oder CO₂C₂H₅ stehen, einen perfluorierten Alkylenrest -(CF₂)ₘ- mit m = 1 bis 10, einen Oxaalkylenrest des Typs -(CH₂)ₙ-O-(CH₂)ₚ-, einen perfluorierten Oxaalkylenrest des Typs -(CF₂)ₙ-O-(CF₂)ₚ- mit n = 1 bis 5 und p = 1 bis 5, oder einen gegebenenfalls perfluorierten Polyoxaalkylenrest mit 2 bis 20 Sauerstoffatomen, die miteinander über mindestens eine -CH₂-, -CF₂- oder -CH₂-CH(CH₃)-Gruppe verbunden sind, für einen Alkylenrest des Typs -(CH₂)ₐ-O-CO-O(CH₂)_{b}-, -(CH₂)ₐ-O-CO-NH-(CH₂)_{b}-, -(CH₂)ₐ-NH-CO-O-(CH₂)_{b}, -(CH₂)ₐ-CO-(CH₂)_{b}- oder -(CH₂)ₐ-O-CO-(CH₂)_{b}-mit a = 1 bis 10 und b = 1 bis 10, einen gegebenenfalls mit Alkyl, OH, OCH₃, OC₂H₅, SH, SCH₃, SC₂H₅, Cl, F, N(Alkyl)₂ oder N(CH₃)C₆H₅ in o-, m- und/oder p-Stellung substituierten Phenylen-, oder einen Cycloalkylenrest mit 5 bis 10 Kohlenstoffatomen, einen (Bis)methylencycloalkylenrest mit 6 bis 12 Kohlenstoffatomen, steht,
- Y: für Wasserstoff, Alkyl oder Aryl, und
- Z: für O oder NY stehen,
oder für den Fall, dass R für einen Arylrest steht, einer der Reste R² oder R⁶ für ein Schwefelatom stehen kann, durch das der Arylrest R in o-Position mit R¹ verbunden ist,
und mit 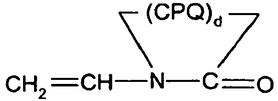 als Formel (VII), wobei
- P, Q:: unabhängig voneinander Wasserstoff und/oder Alkyl und
- d:: eine ganze Zahl von 2 bis 8 bedeuten.

Gegenstand der vorliegenden Erfindung ist ferner ein Verfahren zur Herstellung des erfindungsgemäßen Copolymerisats sowie dessen Verwendung in verschiedensten Anwendungsgebieten, insbesondere zur Porenbildung in und dauerhaften Hydrophilierung von Polymermembranen.

Copolymerisate, enthaltend UV-strahlungsempfindliche Gruppen sind dem Fachmann geläufig (siehe hierzu beispielsweise EP-A 246848, EP-A 377191, EP-A 377199 oder EP-A 1478671). Solche Copolymerisate werden in der Regel durch Polymerisation von Monomeren, welche eine strahlungsempfindliche Gruppe tragen, mit anderen Monomeren, welche im Wesentlichen aus hydrophoben Monomereinheiten, wie Alkylester von α,β-monoethylenisch ungesättigten C₃- bis C₆-Mono- oder Dicarbonsäuren, vinylaromatische Verbindungen, Alkylester von Vinylalkohol, Nitrile α,β-monoethylenisch ungesättigter Carbonsäure oder konjugierte C₄₋₈-Diene aufgebaut sind, hergestellt. Hierbei werden lediglich in untergeordneten Mengen hydrophile Monomere, insbesondere α,β-monoethylenisch ungesättigten C₃- bis C₆-Mono- oder Dicarbonsäuren sowie deren Amide, eingesetzt. In der Regel sind die erhaltenen Polymerisate nicht oder lediglich in sehr eingeschränktem Umfang wasserlöslich, weswegen ihr Einsatz in vielen Anwendungsgebieten nicht möglich ist.

Aufgabe der vorliegenden Erfindung war es daher, im Wesentlichen aus hydrophilen Monomereinheiten aufgebaute und daher gut in Wasser lösliche Copolymerisate, enthaltend strahlungsempfindliche Gruppen zur Verfügung zu stellen.

Überraschender Weise wurde die Aufgabe durch die eingangs definierten Copolymerisate gelöst.

Im Rahmen dieser Schrift steht "Alkyl" für lineare oder verzweigte C₁- bis C₂₄-Alkylreste, bevorzugt C₁- bis C₁₀-Alkylreste und insbesondere für C₁- bis C₄-Alkylreste und "Aryl" für C₆-, C₁₀- oder C₁₄- mono oder polycyclische Aromatenreste, wie Phenyl, 1- oder 2-Naphtyl, 1-, 2- oder 9-Anthracenyl sowie 1-, 2- oder 9-Phenanthryl, welche gegebenenfalls noch mit einem oder mehreren Substituenten, wie insbesondere Hydroxy, Dialkylamino, Halogen, wie Fluor, Chlor und Brom, Alkyoxy, wie Methoxy, Ethoxy und n-Butoxy oder Alkoxycarbonyl, wie Methoxycarbonyl, Ethoxycarbonyl und n-Butoxycarbonyl substituiert sein können. Bevorzugte Alkylreste sind Methyl, Ethyl, n-Propyl, iso-Propyl, n-Butyl, iso-Butyl, tert.-Butyl oder 2-Ethoxyethyl und bevorzugte Arylreste sind Phenyl, p-Chlorophenyl und Tolyl.

Als Monomere A finden Verbindungen der allgemeinen Formel (I) Verwendung, wobei
- R: für einen geradkettigen C₁-C₄-Alkylrest, vorzugsweise Methyl, Ethyl oder n-Propyl, einen verzweigten, gegebenenfalls substituierten C₃- bzw. C₄-Alkylrest, wie iso-Propyl, 2-Hydroxyisopropyl, 2-Dimethylaminoisopropyl, 2-Morpholinoisopropyl oder tert.-Butyl, einen Arylrest, wie beispielsweise Phenyl, o-, m- oder p-Tolyl, 1- oder 2-Naphthyl, oder den Rest R¹ steht und
- R¹: für den Rest 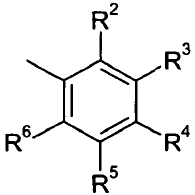
steht, wobei die Reste R² bis R⁶ untereinander gleich oder verschieden sind und für Wasserstoff, Alkyl, Aryl, OH, OCH₃, OC₂Hs, SH, SCH₃, SC₂H₅, F, Cl, Br, CN, CO₂H, CO₂Alkyl, CO₂Aryl, CF₃, N(Alkyl)₂, N(Alkyl)(Aryl), N(Aryl)₂, N⁺(Alkyl)₃A⁻, N⁺H(Alkyl)₂A⁻, wobei A⁻ für das Anion einer Säure, beispielsweise Cl⁻, SO₄²⁻, PO₄³⁻, CH₃CO₂⁻, BF₄⁻, CF₃SO₃⁻, SbF₆⁻, AsF₆⁻ oder PF₆⁻ steht und mindestens einer aber maximal drei der Reste R² bis R⁶ für einen der Reste 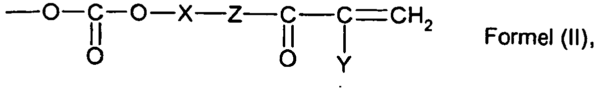 oder 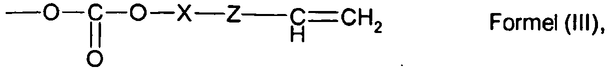 oder 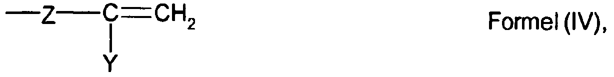 oder 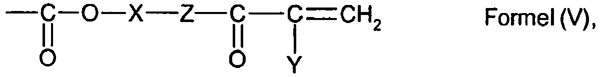 oder 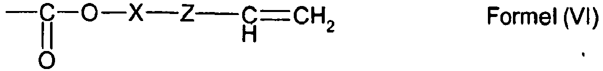 stehen, worin
- X: für einen zweiwertigen, gegebenenfalls substituierten Alkylenrest -(CH₂)ₘ-, einen Rest 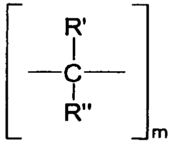
mit m = 1 bis 10, worin R' und R" untereinander gleich oder verschieden sind und für Wasserstoff, Alkyl, Aryl, CO₂H, CO₂CH₃ oder CO₂C₂H₅ stehen, einen perfluorierten Alkylenrest -(CF₂)ₘ- mit m = 1 bis 10, vorzugsweise einen Perfluorethylenrest, einen Oxaalkylenrest des Typs -(CH₂)ₙ-O-(CH₂)ₚ-, einen perfluorierten Oxaalkylenrest des Typs -(CF₂)ₙ-O-(CF₂)ₚ- mit n = 1 bis 5 und p = 1 bis 5, beispielsweise Tetrafluorethylen, oder einen gegebenenfalls perfluorierten Polyoxaalkylenrest mit 2 bis 20 Sauerstoffatomen, die miteinander über mindestens eine -CH₂-, -CF₂- oder -CH₂-CH(CH₃)-Gruppe verbunden sind, für einen Alkylenrest des Typs -(CH₂)ₐ-O-CO-O(CH₂)_{b}-, -(CH₂)ₐ-O-CO-NH-(CH₂)_{b}-, - (CH₂)ₐ-NH-CO-O-(CH₂)_{b}-, -(CH₂)ₐ-CO-(CH₂)_{b}- oder -(CH₂)ₐ-O-CO-(CH₂)_{b}-mit a = 1 bis 10 und b = 1 bis 10, einen gegebenenfalls mit Alkyl, OH, OCH₃, OC₂H₅, SH, SCH₃, SC₂H₅, Cl, F, N(Alkyl)₂ oder N(CH₉)C₆H₅ in o-, m- und/oder p-Stellung substituierten Phenylen-, oder einen Cycloalkylenrest mit 5 bis 10 Kohlenstoffatomen, einen (Bis)methylencycloalkylenrest mit 6 bis 12 Kohlenstoffatomen, steht,
- Y: für Wasserstoff, Alkyl oder Aryl, und
- Z: für O oder NY stehen,
oder für den Fall, dass R für einen Arylrest steht, einer der Reste R² oder R⁶ für ein Schwefelatom stehen kann, durch das der Arylrest R in o-Position mit R¹ verbunden ist.

Die Herstellung dieser Monomeren A ist dem Fachmann beispielsweise aus der EP-A 377191 [wenigstens einer der Reste R² bis R⁶ steht für das Strukturelement der Formein (II) und (III), der EP-A 1478671 [wenigstens einer der Reste R² bis R⁶ steht für das Strukturelement der Formeln (IV)] bekannt. Die in diesen Schriften offenbarten, den Monomeren A entsprechenden Verbindungen, sollen durch diese ausdrückliche Bezugnahme als Bestandteil dieser Schrift angesehen werden. Desweiteren können derartige Monomeren A beispielsweise als Verkaufsprodukte Uvecryl^{®} P 36 der Fa. Sigma-Aldrich Chemie GmbH oder Ebecryl^{®} P 36 der Fa. Cytec Surface Specialties Inc. [wenigstens einer der Reste R² bis R⁶ steht für das Strukturelement der Formel (V)] bezogen werden. Als Monomere A bevorzugt sind die den Monomeren A entsprechenden Verbindungen der Beispiele 19 bis 34 der EP-A 377191, 4-Vinyloxybenzophenon der EP-A 1478671 sowie die vorgenannten Verkaufsprodukte Uvecryl^{®} P 36 und Ebecryl^{®} P 36. Als Monomere A insbesondere bevorzugt sind Verbindungen der allgemeinen Formel (I), in der für
- R: Phenyl,
- R², R³, R⁵, R⁶,: Wasserstoff, und
- R⁴: eine Gruppe der Formel (II) steht, worin
- X: (CH₂)₄,
- Y: Wasserstoff, und
- Z: O,
bedeuten,
oder in der für
- R: Phenyl,
- R², R³, R⁵, R⁶,: Wasserstoff, und
- R⁴: eine Gruppe der Formel (IV) steht, worin
- Y: Wasserstoff, und
- Z: O
bedeuten,
oder in der für
- R: p-Chlorphenyl,
- R², R³, R⁵, R⁶,: Wasserstoff, und
- R⁴: eine Gruppe der Formel (V) steht, worin
- X: (CH₂)₂
- Y: Wasserstoff, und
- Z: O
bedeuten.

Die Gesamtmenge an Monomeren A im erfindungsgemäßen Copolymerisat beträgt 0,01 bis 20 Gew.-%, bevorzugt 0,1 bis 10 Gew.-% und insbesondere bevorzugt 0,1 bis 5 Gew.-% in einpolymerisierter Form.

Als Monomere B finden N-Vinyllactame der allgemeinen Formel (VII) Verwendung, 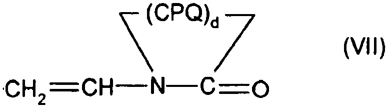 wobei
- P, Q:: unabhängig voneinander Wasserstoff und/oder Alkyl und
- d:: eine ganze Zahl von 2 bis 8 bedeuten.

Dabei können P und Q unabhängig voneinander Wasserstoff und/oder C₁-C₈-Alkyl, beispielsweise Methyl, Ethyl, n-Propyl, iso-Propyl, n-Butyl, iso-Butyl, tert.-Butyl sowie n-Pentyl, n-Hexyl, n-Heptyl oder n-Octyl und deren isomeren Alkylgruppen sein. Als P und Q bevorzugt sind Wasserstoff und Methyl. Insbesondere bevorzugt ist Wasserstoff. Häufig enthält das N-Vinyllactam (VII) keine oder insgesamt lediglich eine Methylgruppe.

Erfindungsgemäß steht d für eine ganze Zahl von 2 bis 8, häufig für 3, 4, 5, 6 und 7. Insbesondere steht d für 3 und 5.

Beispiele für Monomere B sind die N-Vinylderivate nachfolgender Lactame: 2-Pyrrolidon, 2-Piperidon, ε-Caprolactam und deren Alkylderivate, wie beispielsweise 3-Methyl-2-pyrrolidon, 4-Methyl-2-pyrrolidon, 5-Methyl-2-pyrrolidon, 3-Ethyl-2-pyrrolidon, 3-Propyl-2-pyrrolidon, 3-Butyl-2-pyrrolidon, 3,3-Dimethyl-2-pyrrolidon, 3,5-Dimethyl-2-pyrrolidon, 5,5-Dimethyl-2-pyrrolidon, 3,3,5-Trimethyl-2-pyrrolidon, 5-Methyl-5-ethyl-2-pyrrolidon, 3,4,5-Trimethyl-2-pyrrolidon, 3-Methyl-2-piperidon, 4-Methyl-2-piperidon, 5-Methyl-2-piperidon, 6-Methyl-2-piperidon, 6-Ethyl-2-piperidon, 3,5-Dimethyl-2-piperidon, 4,4-Dimethyl-2-piperidon, 3-Methyl-ε-caprolactam, 4-Methyl-ε-caprolactam, 5-Methyl-ε-caprolactam, 6-Methyl-ε-caprolactam, 7-Methyl-ε-caprolactam, 3-Ethyl-ε-caprolactam, 3-Propyl-ε-caprolactam, 3-Butyl-ε-caproiactam, 3,3-Dimethyl-ε-caprolactam oder 7,7-Dimethyl-ε-caprolactam. Selbstverständlich können auch mehrere Monomere B in einpolymerisierter Form vorliegen. Mit besonderem Vorteil finden N-Vinylpyrrolidon und/oder N-Vinylcaprolactam Verwendung, wobei N-Vinylpyrrolidon insbesondere bevorzugt ist.

Die Gesamtmenge an Monomeren B im erfindungsgemäßen Copolymerisat beträgt 50 bis 99,99 Gew.-%, bevorzugt 90 bis 99,9 Gew.-% und insbesondere bevorzugt 95 bis 99,9 Gew.-% in einpolymerisierter Form.

Als Monomere C kommen alle diejenigen ethylenisch ungesättigten Verbindungen in Betracht, welche mit den Monomeren A und B in einfacher Weise radikalisch copolymerisierbar sind, wie beispielsweise vinylaromatische Monomere, wie Styrol, α-Methylstyrol, o-Chlorstyrol oder Vinyltoluole, Vinylhalogenide, wie Vinylchlorid oder Vinylidenchlorid, Ester aus Vinylalkohol und 1 bis 18 C-Atome aufweisenden Monocarbonsäuren, wie Vinylacetat, Vinylpropionat, Vinyl-n-butyrat, Vinylneodecanoat, Vinyllaurat und Vinylstearat, Ester aus vorzugsweise 3 bis 6 C-Atome aufweisenden α,β-monoethylenisch ungesättigten Mono- und Dicarbonsäuren, wie insbesondere Acrylsäure, Methacrylsäure, Maleinsäure, Fumarsäure und Itaconsäure, mit im allgemeinen 1 bis 12, vorzugsweise 1 bis 8 und insbesondere 1 bis 4 C-Atome aufweisenden Alkanolen, wie besonders Acrylsäure- und Methacrylsäuremethyl-, -ethyl-, -n-butyl-, -iso-butyl-, pentyl-, -hexyl-, -heptyl-, -octyl-, -nonyl-, -decyl- und -2-ethylhexylester, Fumar-und Maleinsäuredimethylester oder-di-n-butylester, Nitrile α,β-monoethylenisch ungesättigter Carbonsäuren, wie Acrylnitril, Methacrylnitril, Fumarsäuredinitril, Maleinsäuredinitril, Ether aus Vinylalkohol und 1 bis 18 Kohlenstoffatome aufweisenden Alkoholen, wie beispielsweise n-Butyl-, Cyclohexyl-, Dodecyl-, Ethyl-, 4-Hydroxybutyl- oder Octadecylvinylether sowie C₄₋₈-konjugierte Diene, wie 1,3-Butadien (Butadien) und Isopren. In aller Regel weisen diese Monomeren in Wasser bei Normalbedingungen [20 °C, 1 atm (absolut)] lediglich eine mäßige bis geringe Löslichkeit auf. Im Normalfall werden die vorgenannten Monomeren C lediglich als modifizierende Monomere in Mengen von ≤ 10 Gew.-%, bevorzugt ≤ 5 Gew.-% und insbesondere bevorzugt ≤ 3 Gew.-%, jeweils bezogen auf die Gesamtmenge an Monomeren C, eingesetzt. Insbesondere bevorzugt finden jedoch keinerlei derartigen Monomere C Verwendung.

Monomere C, die unter den vorgenannten Bedingungen eine erhöhte Wasserlöslichkeit aufweisen, sind solche, die entweder wenigstens eine Carbonsäure- oder Sulfonsäuregruppe bzw. deren entsprechendes Anion und/oder wenigstens eine Amino-, Amido-, Ureido- oder N-heterocyclische Gruppe und/oder deren am Stickstoff protonierten oder alkylierten Ammoniumderivate enthalten. Beispielhaft genannt seien Acrylsäure, Methacrylsäure, Fumarsäure, Maleinsäure und Itaconsäure, Vinylsulfonsäure, 2-Acrylamido-2-methylpropansulfonsäure, Styrolsulfonsäure und deren wasserlösliche Salze, vorteilhaft deren Alkalimetall- oder Ammoniumsalze, Acrylamid und Methacrylamid sowie 2-Vinylpyridin, 4-Vinylpyridin, 2-Vinylimidazol, 2-(N,N-Dimethylamino)ethylacrylat, 2-(N,N-Dimethylamino)ethylmethacrylat, 2-(N,N-Diethylamino)ethylacrylat, 2-(N,N-Diethylamino)ethylmethacrylat, 2-(N-tert.-Butylamino)ethylmethacrylat, N-(3-N',N'-Dimethylaminopropyl)methacrylamid und 2-(1-Imidazolin-2-onyl)ethylmethacrylat. Die genannten Monomere bilden in der Regel die Hauptmonomeren, die, bezogen auf die Gesamtmenge an Monomeren C, einen Anteil von ≥ 50 Gew.-%, bevorzugt ≥ 80 Gew.-% und insbesondere bevorzugt ≥ 90 Gew.-% auf sich vereinen oder sogar die Gesamtmenge der Monomeren C bilden. Insbesondere bevorzugt finden jedoch keinerlei derartigen Monomere C Verwendung.

Monomere C, die üblicherweise die innere Festigkeit der Verfilmungen einer Polymermatrix erhöhen, weisen normalerweise wenigstens eine Hydroxy-, wenigstens eine Epoxy-, bzw. wenigstens eine Carbonylgruppe oder wenigstens zwei nicht konjugierte ethylenisch ungesättigte Doppelbindungen auf. Beispiele hierfür sind zwei Vinylreste aufweisende Monomere, zwei Vinylidenreste aufweisende Monomere sowie zwei Alkenylreste aufweisende Monomere. Besonders vorteilhaft sind dabei die Di-Ester zweiwertiger Alkohole mit α,β-monoethylenisch ungesättigten Monocarbonsäuren unter denen die Acryl- und Methacrylsäure bevorzugt sind. Beispiele für derartige zwei nicht konjugierte ethylenisch ungesättigte Doppelbindungen aufweisende Monomere sind Alkylenglykoldiacrylate und -dimethacrylate, wie Ethylenglykoldiacrylat, 1,2-Propylenglykoldiacrylat, 1,3-Propylenglykoldiacrylat, 1,3-Butylenglykoldiacrylat, 1,4-Butylenglykoldiacrylate und Ethylenglykoldimethacrylat, 1,2-Propylenglykoldimethacrylat, 1,3-Propylenglykoldimethacrylat, 1,3-Butylenglykoldimethacrylat, 1,4-Butylenglykoldimethacrylat sowie Divinylbenzol, N,N'-Divinylethylenharnstoff, Vinylmethacrylat, Vinylacrylat, Allylmethacrylat, Allylacrylat, Diallylmaleat, Diallylfumarat, Methylenbisacrylamid, Cyclopentadienylacrylat, Triallylcyanurat oder Triallylisocyanurat. Vorteilhaft können auch hydroxyfunktionalisierte Acryl- oder Methacrylsäurealkylester, wie beispielsweise 2-Hydroxyethyl-, 2-Hydroxypropyl- oder 4-Hydroxybutylacrylat oder -methacrylat eingesetzt werden. Häufig werden die vorgenannten vernetzenden Monomeren C in Mengen von ≤ 10 Gew.-%, bevorzugt jedoch in Mengen von ≤ 3 Gew.-%, jeweils bezogen auf die Gesamtmenge an Monomeren C, eingesetzt. Insbesondere bevorzugt finden jedoch keinerlei derartige Monomere C Verwendung.

Die Gesamtmenge an Monomeren C im erfindungsgemäßen Copolymerisat beträgt 0 bis 49,99 Gew.-%, bevorzugt 0 bis 10 Gew.-% und insbesondere bevorzugt 0 bis 5 Gew.-% in einpolymerisierter Form.

Die Herstellung der erfindungsgemäßen Copolymerisate ist unkritisch und erfolgt durch Polymerisation, bevorzugt durch radikalische Polymerisation der Monomeren A und B sowie gegebenenfalls C. Dabei kann die radikalische Polymerisation der Monomeren A und B sowie gegebenenfalls C in Form einer Substanz- oder in Form einer Lösungspolymerisation erfolgen.

Bei der Herstellung der erfindungsgemäßen Copolymerisate ist es möglich, jeweils gegebenenfalls eine Teil- oder die Gesamtmenge an Monomeren A und B sowie gegebenenfalls C im Polymerisationsgefäß vorzulegen. Es ist aber auch möglich, jeweils die Gesamtmenge oder die gegebenenfalls verbliebene Restmenge an Monomeren A und B sowie gegebenenfalls C während der Polymerisationsreaktion zuzudosieren. Die jeweilige Gesamtmenge oder die gegebenenfalls verbliebene Restmenge an Monomeren A und B sowie gegebenenfalls C kann dem Polymerisationsgefäß dabei diskontinuierlich in einer oder mehreren Portionen oder kontinuierlich mit gleichbleibenden oder sich verändernden Mengenströmen zudosiert werden. Selbstverständlich sind erfindungsgemäß auch Copolymerisate umfasst, welche nach der sogenannten Stufen- bzw. Gradientenfahrweise erhalten werden. Mit Vorteil werden wenigstens Teilmengen der Monomeren vorgelegt.

Mit Vorteil erfolgt die radikalische Polymerisation in Form einer Lösungsmittelpolymerisation beispielsweise in Wasser oder in einem organischen Lösungsmittel. Bevorzugt erfolgt die radikalisch initiierte Lösungspolymerisation der Monomeren A, B und/oder C in einem protischen oder einem aprotischen organischen Lösungsmittel, wobei protische Lösungsmittel insbesondere bevorzugt sind. Als protische organische Lösungsmittel kommen alle organischen Lösemittel in Frage, welche unter Polymerisationsbedingungen wenigstens ein ionisierbares Proton im Molekül enthalten. Beispiele für derartige Lösungsmittel sind bevorzugt alle linearen, verzweigten oder cyclischen C₁- bis C₈-Alkohole, wie beispielsweise Methanol, Ethanol, n-Propanol, iso-Propanol, 1-Butanol, 2-Butanol, 2-Methylpropanol-1, 2-Methylpropanol-2, n-Pentanol sowie isomere Verbindungen, n-Hexanol sowie isomere Verbindungen, n-Heptanol sowie isomere Verbindungen oder n-Octanol sowie isomere Verbindungen, Ethylenglykol, Propylenglykol, aber auch Cyclopentanol, Cyclohexanol, 1,2-Cyclopentandiol oder 1,2-Cyclohexandiol sowie die alkoxylierten, insbesondere ethoxylierten bzw. propoxylierten Derivate der vorgenannten Alkohole. Als aprotische organische Lösungsmittel kommen alle organischen Lösemittel in Frage, welche unter Polymerisationsbedingungen kein ionisierbares Proton im Molekül enthalten. Beispiele für derartige Lösungsmittel sind aromatische Kohlenwasserstoffe, wie Toluol, o-, m-, p-Xylol und Isomerengemische sowie Ethylbenzol, lineare oder cyclische aliphatische Kohlenwasserstoffe, wie Pentan, Hexan, Heptan, Octan, Nonan, Dodecan, Cyclohexan, Cyclooctan, Methylcyclohexan, sowie Mischungen der genannten Kohlenwasserstoffe und Benzinfraktionen, welche keine polymerisierbaren Monomeren enthalten, aliphatische oder aromatische Halogenkohlenwasserstoffe, wie Chloroform, Tetrachlorkohlenstoff, Hexachlorethan, Dichlorethan, Tetrachlorethan, Chlorbenzol sowie flüssige C₁- bzw. C₂-Fluorchlorkohlenwasserstoffe, aliphatische C₂₋ bis C₅-Nitrile, wie Acetonitril, Propionitril, Butyronitril oder Valeronitril, lineare oder cyclische aliphatische C₃- bis C₇-Ketone, wie Aceton, Methylethylketon, Methylisobutylketon, 2- bzw. 3-Hexanon, 2-, 3- bzw. 4-Heptanon, Cyclopentanon, Cyclohexanon, lineare oder cyclische aliphatische Ether, wie Diisopropylether, 1,3- oder 1,4-Dioxan, Tetrahydrofuran oder Ethylenglykoldimethylether, Carbonate, wie Diethylcarbonat, Amide, wie Dimethylacetamid sowie Ester aus aliphatischen C₁- bis C₅-Carbonsäuren oder aromatischen Carbonsäuren mit aliphatischen C₁- bis C₅-Alkoholen, wie Ameisensäureethylester, Ameisensäure-n-propylester, Ameisensäureisopropylester, Ameisensäure-n-butylester, Ameisensäureisobutylester, Ameisensäure-tert.-butylester, Ameisensäureamylester, Essigsäuremethylester, Essigsäureethylester, Essigsäure-n-propylester, Essigsäureisopropylester, Essigsäure-n-butylester, Essigsäureisobutylester, Essigsäure-tert.-butylester, Essigsäureamylester Propionsäuremethylester, Propionsäureethylester, Propionsäure-n-propylester, Propionsäureisopropylester, Propionsäure-n-butylester, Propionsäureisobutylester, Propionsäure-tert.-butylester, Propionsäureamylester, Buttersäuremethylester, Buttersäureethylester, Buttersäure-n-propylester, Buttersäureisopropylester, Buttersäure-n-butylester, Buttersäureisobutylester, Buttersäure-tert.-butylester, Buttersäureamylester, Valeriansäuremethylester, Valeriansäureethylester, Valeriansäure-n-propylester, Valeriansäureisopropylester, Valeriansäure-n-butylester, Valeriansäureisobutylester, Valeriansäure-tert.-butylester, Valeriansäureamylester, Benzoesäuremethylester oder Benzoesäureethylester sowie Lactone, wie Butyrolacton, Valerolacton, Caprolacton, N-Methylpyrrolidon oder N-Ethylpyrrolidon.

Vorzugsweise werden jedoch solche protischen organischen Lösungsmittel ausgewählt, in welchen sich die jeweils eingesetzten Radikalinitiatoren gut lösen. Insbesondere werden solche protischen organischen Lösemittel eingesetzt, in denen sich neben den Radikalinitiatoren auch die erfindungsgemäßen Copolymerisate gut lösen. Insbesondere bevorzugt werden solche protischen organischen Lösungsmittel ausgewählt, welche sich zusätzlich in einfacher Weise, beispielsweise durch Destillation, Inertgasstrippung und/oder Wasserdampfdestillation aus der erhaltenen CopolymerisatLösung abtrennen lassen. Bevorzugte Beispiele hierfür sind Methanol, Ethanol, n-Propanol oder iso-Propanol sowie die entsprechenden Alkohol-Wasser-Gemische. Günstig ist es, wenn das Lösungsmittel bei Atmosphärendruck (1 atm =̂ 1,013 bar absolut) einen Siedepunkt ≤ 140 °C, häufig ≤ 125 °C und insbesondere ≤ 100 °C aufweist oder mit Wasser ein niedrig siedendes Wasser/Lösungsmittel-Azeotropgemisch bildet. Selbstverständlich kann auch eine Mischung mehrerer Lösungsmittel eingesetzt werden. Insbesondere bevorzugt erfolgt die Polymerisation jedoch in wässrigem Medium.

Die Menge an Lösungsmittel bei der Herstellung des erfindungsgemäßen Copolymerisats beträgt 1 bis 9900 Gew.-Teile, bevorzugt 100 bis 1900 Gew.-Teile und insbesondere bevorzugt 150 bis 900 Gew.-Teile, jeweils bezogen auf 100 Gew.-Teile Gesamtmonomeren.

Bei der Herstellung der erfindungsgemäßen Copolymerisate ist es möglich, gegebenenfalls eine Teil- oder die Gesamtmenge an Lösungsmittel im Polymerisationsgefäß vorzulegen. Es ist aber auch möglich, die Gesamtmenge oder die gegebenenfalls verbliebene Restmenge an Lösungsmittel während der Polymerisationsreaktion zuzudosieren. Die Gesamtmenge oder die gegebenenfalls verbliebene Restmenge an Lösungsmittel kann dem Polymerisationsgefäß dabei diskontinuierlich in einer oder mehreren Portionen oder kontinuierlich mit gleichbleibenden oder sich verändernden Mengenströmen zudosiert werden. Vorteilhaft wird eine Teilmenge des Lösungsmittels als Polymerisationsmedium vor Initiierung der Polymerisationsreaktion gemeinsam mit der Gesamt-/Teilmenge der Monomeren A und B sowie gegebenenfalls C im Polymerisationsgefäß vorgelegt und die verbliebene Restmenge gemeinsam mit den Monomeren A und B sowie gegebenenfalls C und dem Radikalinitiator während der Polymerisationsreaktion zudosiert.

Wird die Polymerisation der Monomeren A und B sowie gegebenenfalls C in wässrigem Medium durchgeführt, werden häufig so genannte wasserlösliche Radikalinitiatoren, welche der Fachmann üblicherweise bei der radikalisch initiierten wässrigen Emulsionspolymerisation verwendet, eingesetzt. Wird dagegen die Polymerisation der Monomeren in einem organischen Lösungsmittel durchgeführt, werden in der Regel so genannte öllösliche Radikalinitiatoren, welche der Fachmann üblicherweise bei der radikalisch initiierten Lösungspolymerisation verwendet, eingesetzt. Im Rahmen dieser Schrift sollen als wasserlösliche Radikalinitiatoren alle diejenigen Radikalinitiatoren verstanden werden, die bei 20 °C und Atmosphärendruck in entionisiertem Wasser eine Löslichkeit ≥ 1 Gew.-% aufweisen, während unter öllöslichen Radikalinitiatoren alle diejenigen Radikalinitiatoren verstanden werden, die unter vorgenannten Bedingungen eine Löslichkeit < 1 Gew.-% aufweisen. Häufig weisen wasserlösliche Radikalinitiatoren unter vorgenannten Bedingungen eine Wasserlöslichkeit ≥ 2 Gew.-%, ≥ 5 Gew.-%, oder ≥ 10 Gew.-% auf, während öllösliche Radikalinitiatoren häufig eine Wasserlöslichkeit ≤ 0,9 Gew.-%, ≤ 0,8 Gew.-%, ≤ 0,7 Gew.-%, ≤ 0,6 Gew.-%, ≤ 0,5 Gew.- %, ≤ 0,4 Gew.-%, ≤ 0,3 Gew.-%, ≤ 0,2 Gew.-% oder ≤ 0,1 Gew.-% aufweisen.

Bei den wasserlöslichen Radikalinitiatoren kann sich dabei beispielsweise sowohl um Peroxide wie auch um Azoverbindungen handeln. Als Peroxide können prinzipiell anorganische Peroxide, wie Wasserstoffperoxid oder Peroxodisulfate, wie die Mono- oder Di-Alkalimetall- oder Ammoniumsalze der Peroxodischwefelsäure, wie beispielsweise deren Mono- und Di-Natrium-, -Kalium- oder Ammoniumsalze oder organische Hydroperoxide, wie Alkylhydroperoxide, beispielsweise tert.-Butyl-, p-Mentyl- oder Cumylhydroperoxid eingesetzt werden. Als Azoverbindung finden im wesentlichen 2,2'-Azobis-2-(2-imidazolin-2-yl)propyl)dihydrochlorid oder 2,2'-Azobis(amidinopropyl)dihydrochlorid Verwendung.

Als öllösliche Radikalinitiatoren seien beispielhaft genannt Dialkyl- bzw. Diarylperoxide, wie Di-tert.-amylperoxid, Dicumylperoxid, Bis(tert.-butylperoxiisopropyl)benzol, 2,5-Bis(tert.-butylperoxi)-2,5-dimethylhexan, tert.-Butylcumolperoxid, 2,5-Bis(tert.- butylperoxi)-2,5-dimethyl-3-hexen, 1,1-Bis(tert.-butylperoxi)-3,3,5-trimethylcyclohexan, 1,1-Bis(tert.-butylperoxi)cyclohexan, 2,2-Bis(tert.-butylperoxi)butan oder Di-tert.-butylperoxid, aliphatische und aromatische Peroxiester, wie Cumylperoxineodecanoat, 2,4,4-Trimethylpentyl-2-peroxineodecanoat, tert.-Amylperoxineodecanoat, tert.-Butylperoxineodecanoat, tert.-Amylperoxipivalat, tert.-Butylperoxipivalat, tert.-Amylperoxi-2-ethylhexanoat, tert.-Butylperoxi-2-ethylhexanoat, tert.-Butylperoxidiethylacetat, 1,4-Bis(tert.-butylperoxi)cyclohexan, tert.-Butylperoxiisobutanoat, tert.-Butylperoxi-3,5,5-trimethylhexanoat, tert.-Butylperoxiacetat, tert.-Amylperoxibenzoat oder tert.-Butylperoxibenzoat, Dialkanoyl- bzw. Dibenzoylperoxide, wie Diisobutanoylperoxid, Bis(3,5,5-trimethylhexanoyl)peroxid, Dilauroylperoxid, Didecanoylperoxid, 2,5-Bis(2-ethylhexanoylperoxi)-2,5-dimethylhexan oder Dibenzoylperoxid, sowie Peroxicarbonate, wie Bis(4-tert.-butylcyclohexyl)peroxidicarbonat, Bis(2-ethylhexyl)peroxidicarbonat, Di-tert.-butylperoxidicarbonat, Diacetylperoxidicarbonat, Dimyristylperoxidicarbonat, tert.-Butylperoxiisopropylcarbonat oder tert.-Butylperoxi-2-ethylhexylcarbonat. Als gut öllösliche Azoinitiatoren finden beispielsweise 2,2'-Azobis(isobutyronitril), 2,2'-Azobis(2-methylbutyronitril), Dimethyl-2,2'-azobisisobutyrat, 2,2'-Azobis(2,4-dimethylvaleronitril) und 4,4'-Azobis(4-cyanopentansäure) Verwendung.

Bevorzugt wird als Azo-Radikalinitiator eine Verbindung ausgewählt aus der Gruppe umfassend 2,2'-Azobis(isobutyronitril), 2,2'-Azobis(2-methylbutyronitril), Dimethyl-2,2'-azobisisobutyrat, 2,2'-Azobis(2,4-dimethylvaleronitril), 4,4'-Azobis(4-cyanopentansäure) oder 2,2'-Azobis(amidinopropyl)dihydrochlorid eingesetzt. Selbstverständlich ist es auch möglich, Gemische vorgenannter Radikalinitiatoren einzusetzen.

Die Menge an eingesetztem Radikalinitiator bei der Herstellung des erfindungsgemäβen Copolymerisats beträgt in der Regel 0,01 bis 10 Gew.-%, bevorzugt 0,1 bis 5 Gew.-% und insbesondere bevorzugt 1 bis 4 Gew.-%, jeweils bezogen auf die Gesamtmonomerenmenge.

Bei der Herstellung des erfindungsgemäßen Copolymerisats ist es möglich, gegebenenfalls eine Teil- oder die Gesamtmenge an Radikalinitiator im Polymerisationsgefäß vorzulegen. Es ist aber auch möglich, die Gesamtmenge oder die gegebenenfalls verbliebene Restmenge an Radikalinitiator während der Polymerisationsreaktion zuzudosieren. Die Gesamtmenge oder die gegebenenfalls verbliebene Restmenge an Radikalinitiator kann dem Polymerisationsgefäß dabei diskontinuierlich in einer oder mehreren Portionen oder kontinuierlich mit gleichbleibenden oder sich verändernden Mengenströmen zudosiert werden. Insbesondere vorteilhaft erfolgt die Dosierung der Radikalinitiatoren während der Polymerisationsreaktion kontinuierlich mit gleichbleibendem Mengenstrom - insbesondere in Form einer Lösung des Radikalinitiators mit dem verwendeten Lösungsmittel.

In einer bevorzugten Ausführungsform werden die Gesamtmengen der Monomeren A, B sowie gegebenenfalls C in einem Lösungsmittel vorgelegt und ein radikalischer Polymerisationsinitiator unter Polymerisationsbedingungen diskontinuierlich in mehreren Portionen zudosiert.

Vorteilhaft weist das erfindungsgemäße Copolymerisat einen K-Wert im Bereich ≥ 10 und ≤ 130 (bestimmt bei 25 °C in einer 1 gew.-%igen wässrigen Lösung) auf. Insbesondere vorteilhaft weist das Copolymerisat einen K-Wert im Bereich ≥ 20 und ≤ 100 auf. Die Einstellung des K-Wertes bei der Herstellung des erfindungsgemäßen Copolymerisats ist dem Fachmann geläufig und erfolgt vorteilhaft durch radikalisch initiierte Lösungspolymerisation in Anwesenheit von radikalkettenübertragenden Verbindungen, den sogenannten Radikalkettenreglern. Auch die Bestimmung des K-Wertes ist dem Fachmann geläufig (siehe beispielsweise H. Fikentscher, "Systematik der Cellulosen aufgrund ihrer Viskosität in Lösung" in Cellulose-Chemie 13 (1932), Seiten 58 bis 64 sowie Seiten 71 bis 74 oder Encyclopedia of Chemical Technology, Vol. 21, 2. Ausgabe (1970), Seiten 427 und 428).

Geeignete Radikalkettenregler sind beispielsweise iso-Propanol oder Schwefel in gebundener Form enthaltende organische Verbindungen. Hierzu gehören beispielsweise Mercaptoverbindungen, wie Mercaptoethanol, Mercaptopropanol, Mercaptobutanol, Mercaptoessigsäure, Mercaptopropionsäure, Butylmercaptan und Dodecylmercaptan. Weitere Radikalkettenregler sind dem Fachmann geläufig. Falls die Polymerisation in Gegenwart von Radikalkettenreglern durchgeführt wird, werden häufig 0,01 bis 10 Gew.-%, bezogen auf die Gesamtmonomerenmenge, verwendet.

Bei der Herstellung des erfindungsgemäßen Copolymerisats kann wenigstens eine Teilmenge des Radikalkettenreglers im Polymerisationsmedium vorgelegt werden und die gegebenenfalls verbliebene Restmenge dem Polymerisationsmedium nach Initiierung der radikalischen Polymerisationsreaktion diskontinuierlich in einer Portion, diskontinuierlich in mehreren Portionen sowie kontinuierlich mit gleich bleibenden oder sich verändernden Mengenströmen zugegeben werden.

Durch gezielte Variation von Art und Menge der Monomeren A und B sowie gegebenenfalls C ist es dem Fachmann erfindungsgemäß möglich, Copolymerisate herzustellen, die eine Glasübergangstemperatur bzw. einen Schmelzpunkt im Bereich von -60 bis 270 °C aufweisen. Erfindungsgemäß vorteilhaft beträgt die Glasübergangstemperatur des Copoylmerisats ≥ 40 °C und ≤ 250 °C und bevorzugt ≥ 100 °C und ≤ 200 °C.

Mit der Glasübergangstemperatur T_{g}, ist der Grenzwert der Glasübergangstemperatur gemeint, dem diese gemäß G. Kanig (Kolloid-Zeitschrift & Zeitschrift für Polymere, Bd. 190, S. 1, Gleichung 1) mit zunehmendem Molekulargewicht zustrebt. Die Glasübergangstemperatur bzw. der Schmelzpunkt wird nach dem DSC-Verfahren ermittelt (Dif ferential Scanning Calorimetry, 20 K/min, midpoint-Messung, DIN 53765).

Nach Fox (T.G. Fox, Bull. Am. Phys. Soc. 1956 [Ser. II] 1, Seite 123 und gemäß Ullmann's Encyclopädie der technischen Chemie, Bd. 19, Seite 18, 4. Auflage, Verlag Chemie, Weinheim, 1980) gilt für die Glasübergangstemperatur von höchstens schwach vernetzten Mischpolymerisaten in guter Näherung:

1/Tg = x¹/Tg¹ + x²/Tg² + .... xⁿ/Tg

wobei x¹, x², .... xⁿ die Massenbrüche der Monomeren 1, 2, .... n und T_{g}¹, T_{g}², .... T_{g}ⁿ die Glasübergangstemperaturen der jeweils nur aus einem der Monomeren 1, 2, .... n aufgebauten Polymerisaten in Grad Kelvin bedeuten. Die Tg-Werte für die Homopolymerisate der meisten Monomeren sind bekannt und z.B. in Ullmann's Encyclopedia of Industrial Chemistry, Bd. 5, Vol. A21, Seite 169, VCH Weinheim, 1992, aufgeführt; weitere Quellen für Glasübergangstemperaturen von Homopolymerisaten bilden z.B. J. Brandrup, E.H. Immergut, Polymer Handbook, 1st Ed., J. Wiley, New York 1966, 2nd Ed. J.Wiley, New York 1975, und 3rd Ed. J. Wiley, New York 1989).

Die radikalisch initiierte Polymerisation erfolgt - abhängig vom verwendeten Radikalinitiator - üblicherweise bei Temperaturen im Bereich von 40 bis 180 °C, vorzugsweise von 50 bis 150 °C und insbesondere von 60 bis 110 °C. Sobald die Temperatur bei der Polymerisationsreaktion oberhalb des Siedepunktes des Lösungsmittels und/oder einem der Monomeren A und B sowie gegebenenfalls C liegt, wird die Polymerisation vorteilhaft unter Druck (> 1 atm absolut) durchgeführt. Die Temperatur- und Druckbedingungen sind dem Fachmann geläufig oder können von diesem in wenigen Routineversuchen ermittelt werden.

Die Herstellung des erfindungsgemäßen Copolymerisats kann in Anwesenheit von Luft durchgeführt werden; vorteilhaft erfolgt die Polymerisation jedoch unter sauerstoffarmen oder-freien Bedingungen, wie beispielsweise unter Inertgas, wie Stickstoff oder Argon.

Die Herstellung des erfindungsgemäßen Copolymerisats kann in den üblichen Polymerisationsvorrichtungen erfolgen. Hierzu verwendet man beispielsweise Glaskolben (Labor) oder Rührkessel (technischer Maßstab), die mit einem Anker-, Blatt-, Impeller-, Kreuzbalken-, MIG- oder Mehrstufenimpulsgegenstromrührer ausgestattet sind. Insbesondere bei der Polymerisation in Anwesenheit lediglich kleiner Mengen an Lösungsmittel kann es auch vorteilhaft sein, die Polymerisation in üblichen ein- oder zweiwelligen (gleich- oder gegenläufig) Kneterreaktoren, wie beispielsweise solchen der Fa. List oder Buss SMS durchzuführen.

Erfolgt die Herstellung des erfindungsgemäßen Copolymerisats in einem organischen Lösungsmittel, so wird in der Regel das organische Lösungsmittel zumindest teilweise, vorteilhaft zu ≥ 50 Gew.-% oder ≥ 90 Gew.-% und insbesondere vorteilhaft vollständig entfernt und das Copolymerisat in Wasser, vorteilhaft in entionisiertem Wasser aufgenommen. Die entsprechenden Verfahren sind dem Fachmann geläufig. So kann beispielsweise der Austausch des Lösungsmittels gegen Wasser derart erfolgen, dass das Lösungsmittel, beispielsweise bei Atmosphärendruck oder im Unterdruck (< 1atm absolut) zumindest teilweise, vorteilhaft vollständig in einer oder mehreren Stufen abdestilliert und durch Wasser ersetzt wird. Häufig kann es günstig sein, das Lösungsmittel durch Einleiten von Wasserdampf aus der Lösung zu entfernen und dabei gleichzeitig durch Wasser zu ersetzen. Dies ist insbesondere dann der Fall, wenn das organische Lösungsmittel eine gewisse Wasserdampfflüchtigkeit aufweist.

Insbesondere vorteilhaft sind die erfindungsgemäßen Copolymerisate, die bei 20 °C und 1 atm (absolut) eine Löslichkeit ≥ 100 g, bevorzugt ≥ 300 g und insbesondere bevorzugt ≥ 500 g pro 1000 g entionisiertem Wasser aufweisen. Häufig weisen die Copolymerisate eine unbegrenzte Löslichkeit mit entionisiertem Wasser auf.

Die erfindungsgemäßen Copolymerisate können erfindungsgemäß in Form von Copolymerisatpulvern [beispielsweise aus wässrigen Copolymerisat-Lösungen durch Gefriertrocknung, Walzentrocknung oder Sprühtrocknung zugänglich] oder in Form von Lösungen in wässrigen Medien oder in organischen Lösungsmitteln vorliegen und erfindungsgemäß eingesetzt werden. Mit Vorteil werden Copolymerisatpulver oder Lösungen des erfindungsgemäßen Copolymerisats in einem wässrigen Medium eingesetzt. Insbesondere werden die erfindungsgemäßen Copolymerisate in dem wässrigen Medium eingesetzt, in welchem die Polymerisation der Copolymerisate durchgeführt wurde, oder welches nach Austausch des organischen Lösungsmittels gegen Wasser erhalten wurde.

Die nach dem erfindungsgemäßen Verfahren zugänglichen Copolymerisate wirken einerseits in wässrigem Medium als Verdicker und sind andererseits in der Lage, wasserlösliche bzw. nach UV-Bestrahlung wasserunlösliche Filme auszubilden. Sie werden daher insbesondere in kosmetischen und pharmazeutischen Zubereitungen eingesetzt, beispielsweise als Additive oder Träger in Haarlack, Haarfestiger oder Haarspray, in hautkosmetischen Zubereitungen, als Hautklebegele oder als Immunochemikalien, wie Katheter-Coatings. Spezielle pharmazeutische Anwendungen der erfindungsgemäßen Copolymerisate umfassen insbesondere den Einsatz als Feucht- oder Trockenbindemittel, Matrixretardierungsmittel oder Coatingretardierungsmittel (für Slow-Release-Darreichungsformen), Instant-Release-Coatings und Dragierhilfsmittel. Weiterhin können die erfindungsgemäßen Copolymerisate als Hilfsstoffe für die Agrochemie, beispielsweise für das Saatgut-Coating oder für Soil-Release-Düngemittelformulierungen oder als Hilfsmittel bei der Herstellung von Fischfuttergranulaten verwendet werden.

Aufgrund der hohen Dispergierwirkung der erfindungsgemäßen Copolymerisate, sowohl für organische als auch für anorganische Pigmente, kommen diese als Rostverhinderer oder Rostentfemer von metallischen Oberflächen, als Kesselsteinverhinderer oder Kesselsteinentferner, als Dispergiermittel in Farbstoffpigmentdispersionen, beispielsweise in Druckfarben, in Frage. In diesem Zusammenhang sei auch auf die Verwendung der erfindungsgemäßen Copolymerisate für Tintenstrahl-Aufzeichnungsmedien, Tinten- und Kugelschreiberpasten hingewiesen.

Anwendungstechnisch von Interesse ist auch die hohe Neigung der erfindungsgemäßen Copolymerisate zur Ausbildung von Komplexen mit weiteren organischen Verbindungen, beispielsweise niederen Kohlenwasserstoffen, Phenolen, Tanninen oder diversen Antioxydantien, mit Enzymen und Proteinen sowie mit anderen organischen Polymeren. Weiterhin sind die erfindungsgemäßen Copolymerisate in der Lage, Komplexe mit anorganischen Verbindungen, insbesondere mit Wasserstoffperoxid, Metallen oder Metallsalzen zu bilden. Dementsprechend können die erfindungsgemäßen Copolymerisate vorteilhaft zur Entfernung von Tanninen, Phenolen, Eiweissstoffen oder polyvalenten Kationen aus wässrigem Medium, in Ionenaustauschern, zur Stabilisierung von Wasserstoffperoxid, beispielsweise in Desinfektionsmitteln oder zur Stabilisierung von Antioxydantien, beispielsweise in Konservierungsmitteln verwendet werden. Die erfindungsgemäßen Copolymerisate können weiterhin für die Stabilisierung von Metallkolloiden verwendet werden. In diesem Zusammenhang sei auch auf die Verwendung der erfindungsgemäßen Copolymerisate zur Herstellung von Edelmetall-Kristallisationskeimen für die Silberfällung sowie als Stabilisator für Silberhalogenidemulsionen hingewiesen.

Die erfindungsgemäßen Copolymerisate eignen sich weiterhin zur Modifizierung von Oberflächen- und Grenzflächeneigenschaften. Sie lassen sich daher beispielsweise zur Hydrophilisierung von Oberflächen einsetzen und können so als Textilhilfsmittel, beispielsweise als Abzieh- und Egalisierungshilfsmittel in Textileinfärbungsverfahren oder als Aufhellmittel in Textildruckverfahren verwendet werden. Aufgrund der modifizierenden Wirkung für Oberflächen können die erfindungsgemäßen Copolymerisate zur Beschichtung von organischen und anorganischen Substraten, beispielsweise für Polyolefine, für Glas und Glasfasern verwendet werden. Auch sei in diesem Zusammenhang auf die Verwendung der erfindungsgemäßen Copolymerisate als Hilfsmittel bei der Erdölgewinnung aus ölhaltigem Wasser, als Hilfsmittel bei der Erdöl- und Erdgasförderung sowie dem Erdöl- und Erdgastransport hingewiesen. Weiterhin finden die erfindungsgemäßen Copolymerisate Verwendung als Hilfsmittel bei der Reinigung von Abwässern, sei es als Flockungshilfsmittel oder bei der Entfernung von Farb- und Ölresten aus Abwasser. Auch können die erfindungsgemäßen Copolymerisate als Phasentransferkatalysatoren und als Löslichkeitsverbesserer verwendet werden.

Darüber hinaus finden die erfindungsgemäßen Copolymerisate Verwendung bei der Anfärbung von Polyolefinen, als Farbübertragungsinhibitoren in Waschmitteln, als Farbmischungsinhibitoren für fotografische Diffusions-Transfer-Materialien, als Haftvermittler für Farbstoffe, als Hilfsmittel für die Lithografie, für das Foto-Imaging, für die Diazotypie, als Hilfsmittel für den Metallguss und die Metallhärtung, als Hilfsmittel in Metallquenchbädern, als Hilfsmittel bei der Gasanalytik, als Bestandteil in Keramikbindern, als Papierhilfsmittel für Spezialpapiere, als Bindemittel in Papierstreichfarben und als Bindemittelbestandteil in Gipsbinden.

Die erfindungsgemäßen Copolymerisate eignen sich weiterhin als Protonenleiter und können in elektrisch leitenden Schichten, beispielsweise bei Charge-Transfer-Kathoden, als Festelektrolyte, beispielsweise in Festbatterien, wie insbesondere Lithiumbatterien, eingesetzt werden. Aus den erfindungsgemäßen Copolymerisaten lassen sich auch Kontaktlinsen, synthetische Fasern, Luftfilter, z. B. Zigarettenfilter, oder Membrane hergestellen. Daneben finden die erfindungsgemäßen Copolymerisate Verwendung in wärmebeständigen Schichten, wärmeempfindlichen Schichten und wärmeempfindlichen Widerständen.

Die erfindungsgemäßen Copolymerisate eignen sich besonders vorteilhaft für Anwendungen, bei denen eine photochemische Vernetzung der Copolymerisate von Vorteil sind. Diese Vorteile können beispielsweise die Fixierung an Oberflächen, die Erzeugung von teilvernetzten oder vernetzten Gelen sein. Daher eignen sich die erfindungsgemäßen Copolymerisate und/oder Formulierungen, welche solche Copolymerisate enthalten, vorteilhaft für die Anwendung von Coatingretardierungsmittel und Gelbildner, inbesondere für Slow-Release-Darreichungsformen von Arzneimitteln, für Saatgutbeschichtung, für photochemisch vernetzbare Kleber und Klebegele, insbesondere Hautklebegele, für Tintenstrahl-Aufzeichnungsmedien, Druckfarben, Beschichtungsmittel, beispielsweise für Fasern, Folien, Gläsern und Oberflächen jeglicher Art, zur photochemischen Fixierung der Copolymerisate in Membranen, im besonderen in Polymermembranen und insbesondere zur dauerhaften und nicht auswaschbaren Hydrophilierung von Membranoberflächen.

Insbesondere vorteilhaft finden die erfindungsgemäßen Copolymerisate als Beschichtungsmittel oder Beize für Saatgut, als Bindemittel oder Beschichtungsmittel für Pharmazeutika, als Filmbildner oder Verdicker in Kosmetikformulierungen, als Klebstoffkomponente in Klebstoffen oder Klebstoffstiften, als Beschichtungsmittel von Papier, Glasfasern oder medizinischen Kathetern, als Bindemittel bei der Herstellung von Farben und Lacken sowie zur Porenbildung in und dauerhaften Hydrophilierung von Polymermembranen Verwendung.

Von den erfindungsgemäßen Copolymerisaten können Copolymerisatfilme hergestellt werden, indem Copolymerisat-Lösungen auf Substrate auf oder in Substrate eingebracht werden, danach die Lösungsmittel, beispielsweise durch Erwärmen, bei Normaldruck oder im Unterdruck entfernt und dabei die Copolymerisate bei einer Temperatur, welche höher als die Glasübergangstemperatur bzw. die entsprechende Filmbildungstemperatur der Copolymerisate ist, verfilmt werden und danach die erhaltenen Copolymerisatfilme mit UV-Licht bestrahlt werden, wodurch sich vemetzte Copolymerisatfilme mit neuen, verbesserten Eigenschaften, wie beispielsweise verminderte Wasserlöslichkeit, erhöhte Härte oder verbesserte Dehnungseigenschaften, ausbilden. Als UV-Strahler können die üblichen Strahler, beispielsweise Quecksilberdampfnieder-, - mittel- oder -hochdrucklampen eingesetzt werden, welche Leistungen von 20 bis 100 J/sec x cm² haben können. Dabei ermöglichen Lampen mit höherer Leistung im Allgemeinen eine schnellere Vernetzung. In manchen Fällen kann bei der vernetzenden Bestrahlung gleichzeitig durch den IR-Anteil der Lampen restliches Lösungsmittel entfernt werden.

Sollen die erfindungsgemäßen Copolymerisate zur Porenbildung in und dauerhaften Hydrophilierung von Polymermembranen eingesetzt werden, so werden insbesondere solche synthetische Polymerisate eingesetzt, welche eine gute Löslichkeit in polaren, aprotischen Lösungsmitteln aber keine oder lediglich eine sehr geringe Löslichkeit in wässrigen Medien aufweisen, wie beispielsweise Polysulfone, wie Polyethersulfone, insbesondere polymere aromatische Polysulfone, die beispielsweise unter dem Handelsnamen Ultrason^{®} von BASF SE, Radel^{®} und Udel^{®} von Solvay GmbH vertrieben werden. Daneben werden beispielsweise auch Polyvinylidenflourid (PVDF), welche beispielsweise unter dem Handelsnamen Kynar^{®} PVDF von Arkema S.A., Dyneon^{®} PVDF von Dyneon GmbH oder Solef^{®} PVDF von Solvay GmbH vertrieben werden, sowie geeignete Polycarbonate, Polyvinylchlorid, Polyacrylate, Polyether, Cellulosederivate, Polysorbate, Polyurethane für die Membranherstellung eingesetzt. Dabei erfolgt die Membranherstellung dergestalt, dass das entsprechende synthetische Polymerisat gemeinsam mit einem erfindungsgemäßen Copolymerisat in einem polaren aprotischen Lösungsmittel, insbesondere Dimethylformamid, Dimethylsulfoxid, Dimethylacetamid, N-Methylpyrrolidon oder Gemische daraus gelöst und aus diesen unter geeigneten Fällbedingungen unter Ausbildung poröser Polymermembranen ausgefällt werden.

Dabei werden die Mengen so gewählt, dass in der fertigen Polymerlösung die Menge an synthetischem Polymerisat in der Regel 10 bis 25 Gew.-%, vorzugsweise 12 bis 20 Gew.-% und insbesondere 14 bis 18 Gew. -%, während die Menge an erfindungsgemäßem Copolymerisat in der Regel 0,1 bis 15 Gew.-%, vorzugsweise 1 bis 10 Gew.-% und insbesondere 2 bis 8 Gew.-% beträgt. Neben dem erfindungsgemäßen Copolymerisat können noch andere wasserlösliche Copolymerisate, wie beispielsweise Homooder Copolymerisate des Vinylpyrrolidons eingesetzt werden.

Die Polymerlösung wird in der Regel noch filtriert und anschließend in einer dem Fachmann geläufigen Art und Weise, beispielsweise mittels einer sogenannten Fällungsdüse in ein Fällmedium eingetragen (siehe hierzu beispielsweise K. Ohlrogge, K. Ebert, Membranen: Grundlagen, Verfahren und industrielle Anwendungen, Wiley-VCH-Verlag, Weinheim, 2006).

Dabei besteht das Fällmedium in der Regel aus einem Gemisch eines der vorgenannten polaren aprotischen Lösungsmittel mit Wasser, welches die Fällung des synthetischen Polymerisats auslöst, andererseits aber das hydrophile Copolymerisat unter Ausbildung einer porösen Membran teilweise herauslöst. Selbstverständlich kann das Fällmedium übliche Additive zugesetzt enthalten. Abhängig von der Art des Eintrags der Polymerlösung in das Fällmedium werden bevorzugt Flach- und Hohlfasernmembranen erhalten.

Durch anschließende Bestrahlung der erhaltenen porösen Membranen mit UV-Licht, wird das auf der Membranoberfläche verbliebene erfindungsgemäße Copolymerisat einer Vernetzungsreaktion unterworfen, wodurch das vernetzte erfindungsgemäße Copolymerisat wasserunlöslich wird und so dauerhaft auf der Membranoberfläche anhaftet, während sich die hydrophilen Eigenschaften des vernetzten erfindungsgemäßen Copolymerisats praktisch nicht ändern.

Folgende nichteinschränkenden Beispiele sollen die Erfindung erläutern.

### I. Herstellung der Polymerisate

### Polymerisat A

In einen 2 I-Vierhalskolben, ausgestattet mit Ankerrührer, Rückflusskühler und Stickstoffeinleitung, wurden bei 20 bis 25 °C (Raumtemperartur) und unter Stickstoffatmosphäre 410 g N-Vinylpyrrolidon, 870 g entionisiertes Wasser, 1,2 g einer 25 gew.-%igen wässrigen Ammoniaklösung sowie 11,5 g einer 35 gew.%igen Lösung von 4-[(Benzoylphenoxy)carboxy]butylacrylat in Methylethylketon gegeben und das erhaltene Reaktionsgemisch unter Stickstoffatmosphäre auf 75 °C aufgeheizt. Nach Erreichen dieser Temperatur wurden dem Reaktionsgemisch 10,0 g einer 30 gew.-%igen wässrigen Lösung von Wasserstoffperoxid und 0,5 g einer 0,01 gew.-%igen wässrigen Lösung von Kupfer-II-chlorid in einem Schuss zugegeben. Gleichzeitig beginnend wurden dem Polymerisationsgemisch 2,0 g einer 25 gew.-%igen wässrigen Ammoniaklösung innerhalb von 35 Minuten kontinuierlich zudosiert, daran anschließend das Polymerisationsgemisch auf 85 °C aufgeheizt und 25 Minuten bei dieser Temperatur gerührt. Daran anschließend wurden 1,7 g der 30 gew.-%igen wässrigen Lösung von Wasserstoffperoxid und 0,2 g der 0,01 gew.-%igen wässrigen Lösung von Kupfer-II-chlorid in einem Schuss zugegeben und das Polymerisationsgemisch für eine Stunde bei 85 °C gerührt. Daran anschließend wurden 2,9 g der 30 gew.-%igen wässrigen Lösung von Wasserstoffperoxid und 0,2 g der 0,01 gew.-%igen wässrigen Lösung von Kupfer-II-chlorid in einem Schuss zugegeben, das Polymerisationsgemisch für eine weitere Stunde bei 85 °C gerührt und danach auf Raumtemperatur abgekühlt. Man erhielt eine Polymerlösung mit einem Feststoffgehalt von 32 Gew.-%. Das erhaltene Polymerisat wies einen K-Wert von 29 auf.

Der Feststoffgehalt wurde generell bestimmt, indem eine definierte Menge der wässrigen Polymerisatlösung (ca. 1 g) in einem Aluminiumtiegel mit einem Innendurchmesser von ca. 5 cm bei 120 °C in einem Trockenschrank bis zur Gewichtskonstanz getrocknet wurde (ca. 2 Stunden). Es wurden jeweils zwei separate Messungen durchgeführt. Die in den Beispielen angegebenen Werte stellen den Mittelwert der jeweiligen beiden Messergebnisse dar.

Die K-Wertbestimmung nach Fikentscher erfolgte generell bei 25 °C mit einem Gerät der Fa. Schott, Mainz (Kapillare: Mikro-Ostwald; Typ: MO-Ic). Die erhaltenen wässrigen Copolymerisatlösungen wurden dabei derart mit entionisiertem Wasser gemischt, dass die resultierenden homogenen Lösungen einen Polymerisatgehalt von 1,0 Gew.-% aufwiesen.

### Polymerisat B

In einen 2 I-Vierhalskolben, ausgestattet mit Ankerrührer, Rückflusskühler und Stickstoffeinleitung, wurden bei Raumtemperartur und unter Stickstoffatmosphäre 200 g N-Vinylpyrrolidon, 950 g entionisiertes Wasser gegeben und der pH-Wert mittels einer 25 gew.-%igen wässrigen Ammoniaklösung auf 8,5 eingestellt. Daran anschließend wurde die wässrige Vorlage auf 75 °C aufgeheizt. Beim Erreichen einer Temperatur von 70 °C wurden 0,5 g einer 25 gew.-%igen Lösung von 2,2'-Azobis(2-methylbutyronitril) in iso-Propanol in einem Schuss und gleichzeitig beginnend ein Gemisch aus 43,6 g N-Vinylpyrrolidon und 6,4 g einer 35 gew.-%igen Lösung von 4-[(Benzoylphenoxy)carboxy]butylacrylat in Methylethylketon innerhalb von 30 Minuten kontinuierlich zudosiert. Daran anschließend ließ man das Polymerisationsgemisch für 30 Minuten bei 75 °C rühren, gab erneut 0,5 g der isopropanolischen Lösung von 2,2'-Azobis(2-methylbutyronitril) in einem Schuss zu, ließ für weitere 60 Minuten bei 75 °C rühren, gab weitere 2,0 g der isopropanolischen Lösung von 2,2'-Azobis(2-methylbutyronitril) in einem Schuss zu, ließ für weitere 30 Minuten bei 75 °C rühren, heizte dann das Polymerisationsgemisch auf 90 °C auf und beließ es für 2 Stunden bei dieser Temperatur. Die so erhaltene Copolymerisatlösung wurde mit Ameisensäure auf einen pH-Wert von 3 eingestellt, 30 Minuten gerührt, anschließend mittels der 25 gew.-%igen wässrigen Ammoniaklösung neutralisiert und dann die erhaltene Polymerisatlösung so lange mit Wasserdampf gestrippt, bis ca. 500 ml Flüssigkeit aus dem Polymerisationsgefäß in die Destillationsvorlage abdestilliert waren. Anschließend wurde die erhaltene Polymerisatlösung auf Raumtemperatur abgekühlt. Die so erhaltene Polymerisatlösung wies einen Feststoffgehalt von 21 Gew.-% auf. Der K-Wert wurde zu 93 bestimmt.

### Polymerisat C

Die Herstellung von Polymerisat C erfolgte analog zu Polymerisat B, mit dem Unterschied, dass 2,4 g 4-Vinyloxybenzophenon anstelle von 4-[(Benzoylphenoxy)carboxy]butylacrylat zugegeben wurden. Die so erhaltene Polymerisatlösung wies einen Feststoffgehalt von 23 Gew.-% auf. Der K-Wert wurde zu 90 bestimmt.

### Polymerisat D

Die Herstellung von Polymerisat D erfolgte analog zu Polymerisat B, mit dem Unterschied, dass 2,4 g 2-(Acryloyloxy)ethyl-4-(4-chlorobenzoyl)benzoat (Uvecryl^{®} P 36 der Firma Sigma-Aldrich Chemie GmbH) anstelle von 4-[(Benzoylphenoxy)carboxy]butylacrylat zugegeben wurden. Die so erhaltene Polymerisatlösung wies einen Feststoffgehalt von 25 Gew.-% auf. Der K-Wert wurde zu 95 bestimmt. -

### Polymerisat E (Vergleichsbeispiel 1)

Die Herstellung von Polymerisat E erfolgte analog zur Herstellung von Polymerisat A, mit dem Unterschied, dass kein 4-[(Benzoylphenoxy)carboxy]butylacrylat eingesetzt wurde. Die so erhaltene Polymerisatlösung wies einen Feststoffgehalt von 31 Gew.-% auf. Es wurde ein K-Wert von 31 bestimmt.

### Polymerisat F (Vergleichsbeispiel 2)

Die Herstellung von Polymerisat F erfolgte analog zur Herstellung von Polymerisat B, mit dem Unterschied, dass kein 4-[(Benzoylphenoxy)carboxy]butylacrylat eingesetzt wurde. Die so erhaltene Polymerisatlösung wies einen Feststoffgehalt von 19 Gew.-% auf. Es wurde ein K-Wert von 93 bestimmt.

### II. Untersuchungen zur Löslichkeit der Polymerisatfilme

Alle vorgenannten Polymerisatlösungen wurden mit entionisiertem Wasser auf einen Feststoffgehalt von 15 Gew.-% eingestellt. Anschließend wurden Teilmengen der Polymerlösungen der Polymerisate E und F gefriergetrocknet, anschließend in iso-Propanol zu einer 15 gew.-%igen alkoholischen Lösung aufgenommen und mit jeweils 1 Gew.-% der folgenden kommerziell erhältlichen Fotoinitiatoren Irgacur^{®} 500 und Darocur^{®} 1173 (Verkaufsprodukte der Firma Ciba Spezialitätenchemie, Lampertheim) sowie Benzophenon, jeweils bezogen auf den Feststoffgehalt der Polymerisatlösungen, versetzt und durch 5-minütiges Rühren homogen vermischt. Daran anschließend wurden die Polymerisatlösungen der Polymerisate A bis F, sowie der mit den vorgenannten Fotoinititiatoren additivierten Polymerisatlösungen der Polymerisate E und F in zwei Serien mit einer Schichtdicke von 500 µm auf fettfreie 8 x 30 cm-Glasplatten aufgetragen und die so erhaltenen beschichteten Glasplatten für 24 Stunden in einem Klimaraum bei 23 °C und 50 % relativer Luftfeuchtigkeit getrocknet. Die auf den Glasplatten erhaltenen Polymerisatfilme wiesen eine Schichtdicke von ca. 50 µm auf. Nach dem Trocknen wurde eine Serie der beschichteten Glasplatten mittels einer UV-Belichtungsanlage der Firma IST Strahlentechnik Metz GmbH, enthaltend zwei Lampen des Typs eta plus M400-U2HC (UV-Strahler im Wellenlängenbereich von 180 bis 450 nm) mit einer Strahlungsintensität von 3700 mJ/cm² bestrahlt. Daran anschließend wurden die beschichteten Glasplatten beider Serien bei Raumtemperatur für 5 Minuten senkrecht in entionisiertes Wasser getaucht, anschließend die beschichteten Glasplatten mit einem Baumwollgewebe trockengetupft und die Beschichtung der Glasplatten visuell bewertet. Dabei wurden die in folgender Tabelle aufgelisteten Ergebnisse erhalten:

| Polymerisat | Filmzustand nach unbestrahlt | Wasserbad bestrahlt |
|---|---|---|
| A | gelöst | ungelöst |
| B | gelöst | ungelöst |
| C | gelöst | ungelöst |
| D | gelöst | ungelöst |
| E | gelöst | gelöst |
| E + Irgacur 500 | gelöst | gelöst |
| E + Darocur 1173 | gelöst | gelöst |
| E + Benzophenon | gelöst | gelöst |
| F | gelöst | gelöst |
| F + Irgacur 500 | gelöst | gelöst |
| F + Darocur 1173 | gelöst | gelöst |
| F + Benzophenon | gelöst | gelöst |

Aus den Ergebnissen ist klar ersichtlich, dass die erfindungsgemäßen Copolymerisate (Polymerisate A bis D) nach dem Bestrahlen mit UV-Licht wasserunlösliche, teils leicht gequollene Filme ausbildeten, während sich die entsprechenden Polymerisatfilme ohne UV-Bestrahlung vollständig in Wasser auflösten. Dagegen bildeten Polyvinylpyrrolidone (Polymerisate E und F), welche keine Monomere der allgemeinen Formel (I) in einpolymerisierter Form enthielten oder denen sogenannte UV-Fotoinitiatoren als separate Komponenten nachträglich zugemischt wurden, auch nach entsprechender UV-Bestrahlung nur gut wasserlösliche Polymerisatfilme aus.

## Patentansprüche

1. Copolymerisat, enthaltend in einpolymerisierter Form
| | |
|---|---|
| 0,01 bis 20 Gew.-% | wenigstens einer ethylenisch ungesättigten Verbindung der allgemeinen Formel (I) [Monomere A], |
| 50 bis 99,99 Gew.-% | wenigstens eines N-Vinyllactams der allgemeinen Formel (VII) [Monomere B] und |
| 0 bis 49,99 Gew.-% | wenigstens einer weiteren, sich von den Monomeren A und Monomeren B unterscheidenden ethylenisch ungesättigten Verbindung [Monomere C], wobei sich die Gesamtmengen der Monomeren A, B und C zu 100 Gew.-% aufsummieren (Gesamtmonomerenmenge), mit |
R-(C=O)-R¹ als Formel (I), wobei
R für einen geradkettigen C₁-C₄-Alkylrest, einen verzweigten, gegebenenfalls substituierten C₃- bzw. C₄-Alkylrest, einen Arylrest, oder den Rest R¹ steht und
R¹ für den Rest 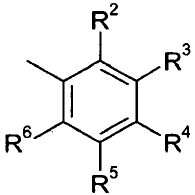
steht, wobei die Reste R² bis R⁶ untereinander gleich oder verschieden sind und für Wasserstoff, Alkyl, Aryl, OH, OCH₃, OC₂H₅, SH, SCH₃, SC₂Hs, F, Cl, Br, CN, CO_{2H}, CO₂Alkyl, CO₂Aryl, CF₃, N(Alkyl)₂, N(Alkyl)(Aryl), N(Aryl)₂, N+(Alkyl)₃A⁻, N⁺H(Alkyl)₂A⁻, wobei A⁻ für das Anion einer Säure steht und mindestens einer aber maximal drei der Reste R² bis R⁶ für einen der Reste 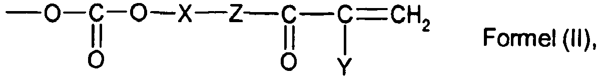 oder 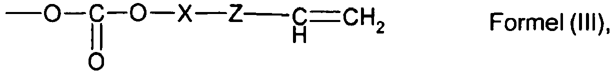 oder 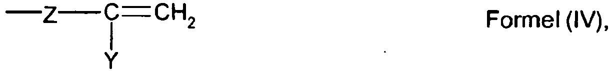 oder 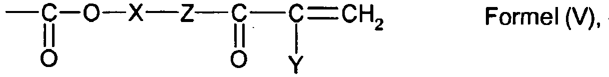 oder 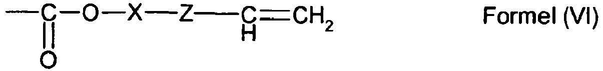 stehen, worin
X für einen zweiwertigen, gegebenenfalls substituierten Alkylenrest -(CH₂)ₘ-, einen Rest 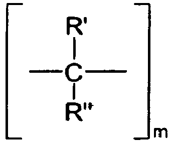 mit m = 1 bis 10, worin R' und R" untereinander gleich oder verschieden sind und für Wasserstoff, Alkyl, Aryl, CO₂H, CO₂CH₃ oder CO₂C₂H₅ stehen, einen perfluorierten Alkylenrest -(CF₂)ₘ- mit m = 1 bis 10, einen Oxaalkylenrest des Typs -(CH₂)ₙ-O-(CH₂)ₚ-, einen perfluorierten Oxaalkylenrest des Typs -(CF₂)ₙ-O-(CF₂)ₚ- mit n = 1 bis 5 und p = 1 bis 5, oder einen gegebenenfalls perfluorierten Polyoxaalkylenrest mit 2 bis 20 Sauerstoffatomen, die miteinander über mindestens eine -CH₂-, -CF₂- oder -CH₂-CH(CH₃)-Gruppe verbunden sind, für einen Alkylenrest des Typs -(CH₂)ₐ-O-CO-O(CH₂)_{b}-, -(CH₂)ₐ-O-CO-NH-(CH₂)_{b}-, -(CH₂)ₐ-NH-CO-O-(CH₂)_{b}-, -(CH₂)ₐ-CO-(CH₂)_{b}- oder -(CH₂)ₐ-O-CO-(CH₂)_{b}- mit a = 1 bis 10 und b = 1 bis 10, einen gegebenenfalls mit Alkyl, OH, OCH₃, OC₂H₅, SH, SCH₃, SC₂H₅, Cl, F, N(Alkyl)₂ oder N(CH₃)C₆H₅ in o-, m- und/oder p-Stellung substituierten Phenylen-, oder einen Cycloalkylenrest mit 5 bis 10 Kohlenstoffatomen, einen (Bis)methylencycloalkylenrest mit 6 bis 12 Kohlenstoffatomen, steht,
Y für Wasserstoff, Alkyl oder Aryl, und
Z für O oder NY stehen,
oder für den Fall, dass R für einen Arylrest steht, einer der Reste R² oder R⁶ für ein Schwefelatom stehen kann, durch das der Arylrest R in o-Position mit R¹ verbunden ist,
und mit 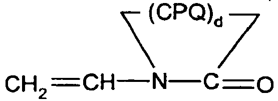 als Formel (VII), wobei
P, Q: unabhängig voneinander Wasserstoff und/oder Alkyl und
d: eine ganze Zahl von 2 bis 8 bedeuten.

2. Copolymerisat nach Anspruch 1, enthaltend ausschließlich
| | |
|---|---|
| 0,1 bis 5 Gew.-% | an Monomeren A und |
| 95 bis 99,9 Gew.-% | an Monomeren B. |

3. Copolymerisat nach einem der Ansprüche 1 oder 2, enthaltend als Monomer B N-Vinylpyrrolidon und/oder N-Vinylcaprolactam.

4. Copolymerisat nach einem der Ansprüche 1 bis 3, enthaltend als Monomer A eine Verbindung in der für
R Phenyl,
R², R³, R⁵, R⁶, Wasserstoff, und
R⁴ eine Gruppe der Formel (II) steht, worin
X (CH₂)₄,
Y Wasserstoff, und
Z O
bedeuten.

5. Copolymerisat nach einem der Ansprüche 1 bis 3, enthaltend als Monomer A eine Verbindung in der für
R Phenyl,
R², R³, R⁵, R⁶, Wasserstoff, und
R⁴ eine Gruppe der Formel (IV) steht, worin
Y Wasserstoff, und
Z O
bedeuten.

6. Copolymerisat nach einem der Ansprüche 1 bis 3, enthaltend als Monomer A eine Verbindung in der für
R p-Chlorphenyl,
R², R³, R⁵, R⁶, Wasserstoff, und
R⁴ eine Gruppe der Formel (V) steht, worin
X (CH₂)₂
Y Wasserstoff, und
Z O
bedeuten.

7. Verfahren zur Herstellung eines Copolymerisats gemäß einem der Ansprüche 1 bis 6, **dadurch gekennzeichnet, dass** die Monomeren A und B sowie gegebenenfalls C radikalisch polymerisiert werden.

8. Verfahren nach Anspruch 7, **dadurch gekennzeichnet, dass** die Gesamtmengen Monomeren A, B sowie gegebenenfalls C in einem Lösungsmittel vorgelegt und ein radikalischer Polymerisationsinitiator unter Polymerisationsbedingungen zudosiert wird.

9. Verwendung eines Copolymerisats gemäß einem der Ansprüche 1 bis 6 als Beschichtungsmittel oder Beize für Saatgut, Bindemittel oder Beschichtungsmittel für Pharmazeutika, Filmbildner oder Verdicker in Kosmetikformulierungen, Klebstoffkomponente in Klebstoffen oder Klebstoffstiften, Beschichtungsmittel von Papier, Glasfasern oder medizinischen Kathetern, Bindemittel bei der Herstellung von Farben und Lacken sowie zur Porenbildung in und dauerhaften Hydrophilierung von Polymermembranen.

10. Verfahren zum Beschichten bzw. zum Modifizieren eines Substrats, **dadurch gekennzeichnet, dass** eine Lösung eines Copolymerisats gemäß einem der Ansprüche 1 bis 6 auf ein Substrat auf oder in ein Substrat eingebracht wird, danach das Lösungsmittel bei Normaldruck oder im Unterdruck entfernt wird, dabei das Copolymerisat verfilmt wird und danach der erhaltenen Copolymerisatfilm mit UV-Licht bestrahlt wird.

## Claims

1. A copolymer comprising in copolymerized form
| | |
|---|---|
| 0.01% to 20% | by weight of at least one ethylenically unsaturated compound of the general formula (I) [monomers A], |
| 50% to 99.99% | by weightof at least one N-vinyllactam of the general formula (VII) [monomers B], and |
| 0% to 49.99% | by weight of at least one further ethylenically unsaturated compound [monomers C] which differs from the monomers A and monomers B, the total amounts of monomers A, B and C summing to 100% by weight (total monomer amount), with |
R-(C=O)-R¹ as formula (I), where
R is a straight-chain C₁-C₄ alkyl radical, a branched, optionally substituted C₃ or C₄ alkyl radical, an aryl radical or the radical R¹, and
R¹ is the radical 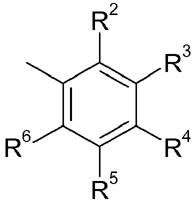
where the radicals R² to R⁶ are like or different from one another and are hydrogen, alkyl, aryl, OH, OCH₃, OC₂H₅, SH, SCH₃, SC₂H₅, F, Cl, Br, CN, CO₂H, CO₂alkyl, CO₂aryl, CF₃, N (alkyl)₂, N (alkyl) (aryl), N(aryl)₂, N⁺(alkyl)₃A⁻, N⁺H (alkyl)₂A⁻, where A⁻ is the anion of an acid, and at least one but not more than three of the radicals R² to R⁶ is or are one of the radicals 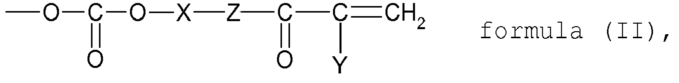
or 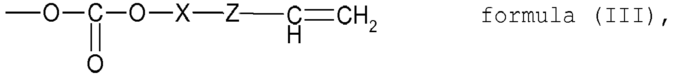 or 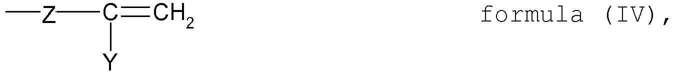 or 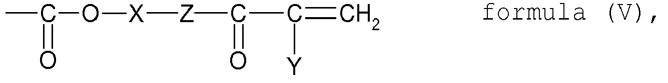 or 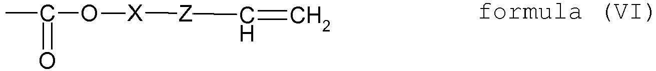 in which
X is a divalent, optionally substituted alkylene radical -(CH₂)ₘ-, a radical 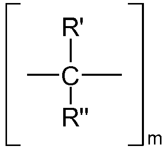
with m = 1 to 10, in which R' and R" are like or different from one another and are hydrogen, alkyl, aryl, CO₂H, CO₂CH₃ or CO₂C₂H₅, a perfluorinated alkylene radical -(CF₂)ₘ- with m = 1 to 10, an oxaalkylene radical of the type - (CH₂)ₙ-O-(CH₂)ₚ-, a perfluorinated oxaalkylene radical of the type - (CF₂)ₙ-O-(CF₂)ₚ- with n = 1 to 5 and p = 1 to 5, or an optionally perfluorinated polyoxaalkylene radical having 2 to 20 oxygen atoms which are joined to one another via at least one -CH₂-, -CF₂- or -CH₂-CH(CH₃)-group, or are an alkylene radical of the type - ( CH₂)ₐ-O-CO-O(CH₂)_{b}-, -(CH₂)ₐ-O-CO-NH-(CH₂)_{b}-, -(CH₂)ₐ-NH-CO-O-(CH₂)_{b}-, -(CH₂)ₐ-CO-(CH₂)_{b}- or -(CH₂)ₐ-O-CO-(CH₂)_{b}- with a = 1 to 10 and b = 1 to 10, a phenylene radical which is optionally substituted by alkyl, OH, OCH₃, OC₂H₅, SH, SCH₃, SC₂H₅, Cl, F, N(alkyl)₂ or N(CH₃)C₆H₅ in o-, m- and/or p-position, or a cycloalkylene radical having 5 to 10 carbon atoms or a (bis)methylenecycloalkylene radical having 6 to 12 carbon atoms,
Y is hydrogen, alkyl or aryl, and
Z is 0 or NY,
or, if R is an aryl radical, one of the radicals R² or R⁶ may be a sulfur atom which connects the aryl radical R in o-position to R¹,
and with 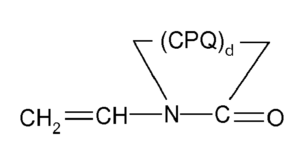 as formula (VII), where
P and Q: independently of one another are
hydrogen and/or alkyl and
d: is an integer from 2 to 8.

2. The copolymer according to claim 1, comprising exclusively
0.1% to 5% by weight of monomers A and
95% to 99.9% by weight of monomers B.

3. The copolymer according to either of claims 1 and 2, comprising N-vinylpyrrolidone and/or N-vinylcaprolactam as monomer B.

4. The copolymer according to any of claims 1 to 3, comprising as monomer A a compound in which
R is phenyl,
R², R³, R⁵, and R⁶ are hydrogen, and
R⁴ is a group of the formula (II) in which
X is (CH₂)₄,
Y is hydrogen, and
Z is O.

5. The copolymer according to any of claims 1 to 3, comprising as monomer A a compound in which
R is phenyl,
R², R³, R⁵, and R⁶ are hydrogen, and
R⁴ is a group of the formula (IV) in which
Y is hydrogen and
Z is O.

6. The copolymer according to any of claims 1 to 3, comprising as monomer A a compound in which
R is p-chlorophenyl,
R², R³, R⁵, and R⁶ are hydrogen, and
R⁴ is a group of the formula (V) in which
X is (CH₂)₂,
Y is hydrogen, and
Z is O.

7. A process for preparing a copolymer according to any of claims 1 to 6, which comprises subjecting the monomers A and B and also optionally C to free-radical polymerization.

8. The process according to claim 7, wherein the total amounts of monomers A, B, and optionally C are introduced in a solvent and a free-radical polymerization initiator is metered in under polymerization conditions.

9. The use of a copolymer according to any of claims 1 to 6 as a coating material or dressing for seed, a binder or coating material for pharmaceuticals, a film-former or thickener in cosmetics formulations, an adhesive component in adhesives or glue sticks, a coating material for paper, glass fibers or medical catheters, a binder in the production of paints and varnishes, or for pore formation in and permanent hydrophilization of polymer membranes.

10. A method of coating and/or modifying a substrate, which comprises introducing a solution of a copolymer according to any of claims 1 to 6 onto a substrate or into a substrate, thereafter removing the solvent under atmospheric pressure or under subatmospheric pressure, the copolymer filming in the course of this procedure, and thereafter irradiating the resulting copolymer film with UV light.

## Revendications

1. Copolymère, contenant sous forme copolymérisée
0,01 à 20 % en poids d'au moins un composé éthyléniquement insaturé de formule générale (I) [monomères A],
50 à 99,99 % en poids d'au moins un N-vinyllactame de formule générale (VII) [monomères B] et
0 à 49,99 % en poids d'au moins un autre composé éthyléniquement insaturé différent des monomères A et des monomères B [monomères C], les quantités totales des monomères A, B et C s'additionnant pour atteindre 100 % en poids (quantité totale de monomères), avec
R-(C=O)-R¹ en tant que formule (I), dans laquelle
R représente un radical alkyle en C₁-C₄ linéaire, un radical alkyle en C₃ ou C₄ ramifié, éventuellement substitué, un radical aryle ou le radical R¹, et
R¹ représente le radical 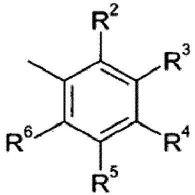
les radicaux R² à R⁶ étant identiques ou différents les uns des autres et représentant hydrogène, alkyle, aryle, OH, OCH₃, OC₂H₅, SH, SCH₃, SC₂H₅, F, Cl, Br, CN, CO₂H, CO₂-alkyle, CO₂-aryle, CF₃, N (alkyle)₂, N(alkyle)(aryle), N(aryle)₂, N⁺(alkyle)₃A⁻, N⁺H(alkyle)₂A⁻, A⁻ représentant l'anion d'un acide et au moins un mais au plus trois des radicaux R² à R⁶ représentant un des radicaux 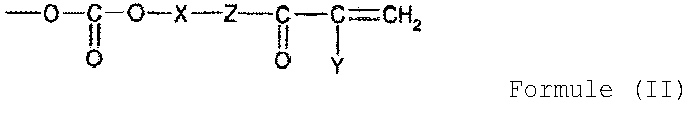 ou 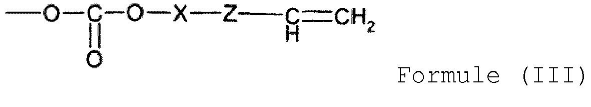 ou 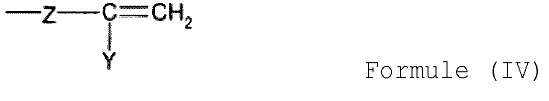 ou 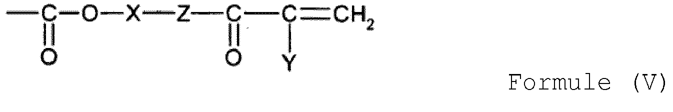 ou 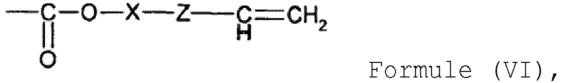 dans lesquelles
X représente un radical alkylène bivalent éventuellement substitué -(CH₂)ₘ-, un radical 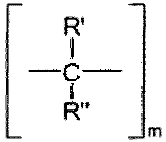 avec m = 1 à 10, R' et R" étant identiques ou différents l'un de l'autre et représentant hydrogène, alkyle, aryle, CO₂H, CO₂CH₃ ou CO₂C₂H₅, un radical alkylène perfluoré - (CF₂)ₘ- avec m = 1 à 10, un radical oxaalkylène de type - (CH₂)ₙ-O-(CH₂)ₚ-, un radical oxaalkylène perfluoré de type - (CF₂)n-O-(CF₂)p- avec n = 1 à 5 et p = 1 à 5, ou un radical polyoxaalkylène éventuellement perfluoré contenant 2 à 20 atomes d'oxygène, qui sont reliés les uns aux autres par au moins un groupe -CH₂-, -CF₂- ou -CH₂-CH(CH₃)-, un radical alkylène de type - (CH₂)ₐ-O-CO-O(CH₂)_{b}-, - (CH₂)ₐ-O-CO-NH-(CH₂)_{b}-, -(CH₂)ₐ-NH-CO-O-(CH₂)_{b}-, -(CH₂)ₐ-CO-(CH₂)_{b}- OU - (CH₂)ₐ-O-CO-(CH₂)_{b}- avec a = 1 à 10 et b = 1 à 10, un radical phénylène ou cycloalkylène contenant 5 à 10 atomes de carbone, éventuellement substitué avec alkyle, OH, OCH₃, OC₂H₅, SH, SCH₃, SC₂H₅, Cl, F, N(alkyle)₂ ou N(CH₃)C₆H₅ en position o, m et/ou p, un radical (bis)méthylènecycloalkylène contenant 6 à 12 atomes de carbone,
Y représente hydrogène, alkyle ou aryle et
Z représente 0 ou NY,
ou, dans le cas où R représente un radical aryle, un des radicaux R² ou R⁶ peut représenter un atome de soufre, par lequel le radical aryle R est relié avec R¹ en position o,
et avec 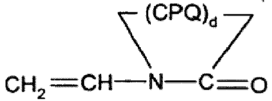 en tant que formule (VII), dans laquelle
P, Q : signifient indépendamment l'un de l'autre hydrogène et/ou alkyle, et
d : signifie un nombre entier de 2 à 8.

2. Copolymère selon la revendication 1, contenant exclusivement
| | |
|---|---|
| 0,1 à 5 % en poids | de monomères A et |
| 95 à 99,9 % en poids | de monomères B. |

3. Copolymère selon l'une quelconque des revendications 1 ou 2, contenant en tant que monomère B de la N-vinylpyrrolidone et/ou du N-vinylcaprolactame.

4. Copolymère selon l'une quelconque des revendications 1 à 3, contenant en tant que monomère A un composé dans lequel
R signifie phényle,
R², R³, R⁵, R⁶ signifient hydrogène et
R⁴ signifie un groupe de formule (II), dans laquelle
X signifie (CH₂)₄,
Y signifie hydrogène et
Z signifie O.

5. Copolymère selon l'une quelconque des revendications 1 à 3, contenant en tant que monomère A un composé dans lequel
R signifie phényle,
R², R³, R⁵, R⁶ signifient hydrogène et
R⁴ signifie un groupe de formule (IV), dans laquelle
Y signifie hydrogène et
Z signifie O.

6. Copolymère selon l'une quelconque des revendications 1 à 3, contenant en tant que monomère A un composé dans lequel
R signifie p-chlorophényle,
R² R³, R⁵, R⁶ signifient hydrogène et
R⁴ signifie un groupe de formule (V), dans
laquelle
X signifie (CH₂)₂,
Y signifie hydrogène et
Z signifie O.

7. Procédé de fabrication d'un copolymère selon l'une quelconque des revendications 1 à 6, **caractérisé en ce que** les monomères A et B et éventuellement C sont polymérisés par voie radicalaire.

8. Procédé selon la revendication 7, **caractérisé en ce que** les quantités totales des monomères A, B et éventuellement C sont chargées initialement dans un solvant et un initiateur de polymérisation radicalaire est ajouté dans des conditions de polymérisation.

9. Utilisation d'un copolymère selon l'une quelconque des revendications 1 à 6 en tant qu'agent de revêtement ou mordant pour semence, liant ou agent de revêtement pour produits pharmaceutiques, agent filmogène ou épaississant dans des formulations cosmétiques, composant adhésif dans des adhésifs ou des bâtons de colle, agent de revêtement de papier, fibres de verre ou cathéters médicaux, liant lors de la fabrication de peintures et de vernis, et pour la formation de pores dans et l'hydrophilisation durable de membranes polymères.

10. Procédé de revêtement ou de modification d'un substrat, **caractérisé en ce qu'**une solution d'un copolymère selon l'une quelconque des revendications 1 à 6 est appliquée sur un substrat ou dans un substrat, puis le solvant est éliminé à pression normale ou sous-pression, le copolymère formant un film, puis le film de copolymère obtenu étant exposé à une lumière UV.
